# EUROPEAN PATENT APPLICATION

(11) **EP 1 532 987 A1**
(43) Date of publication of application: **25.05.2005**
(21) Application number: 03730789.9
(22) Date of filing: 03.06.2003
(51) Int. Cl.: A61K 48/00, A61K 38/27, A61K 9/06, A61K 9/08, A61K 9/107, A61K 9/20, A61K 9/28, A61K 9/48, A61K 31/711, A61P 9/00, A61P 9/10, A61P 25/28, A61P 43/00

(54) **AGENTS FOR GENE THERAPEUTIC FOR CEREBROVASCULAR DISORDERS**

(30) Priority: 06.06.2002 JP 2002165437
(71) Applicant: AnGes MG, Inc., Ibaraki, Osaka 567-0085 (JP)
(72) Inventor: MORISHITA, Ryuichi, Osaka 565-0851 (JP); SHIMAMURA, Munehisa, Minoo-shi, Osaka 562-0031 (JP)
(74) Representative: Woods, Geoffrey Corlett
(86) International application number: PCT/JP2003/007004
(87) International publication number: WO 2003/103721

(57) **Abstract**

The present invention provides novel methods for treating cerebrovascular disorders, in which HGF is overexpressed by introducing an HGF gene. The methods of this invention using an HGF gene enable active treatment of cerebrovascular disorders, such as cerebral infarction, by gene transfer, and enable the maintenance of neuronal function and the suppression of infarcted areas in patients for whom appropriate treatment methods were unavailable until now.

## Description

### Technical Field

The present invention relates to the treatment or prevention of cerebrovascular disorders by using a hepatocyte growth factor (HGF) gene. More specifically, the present invention relates to agents for treatment or prevention, that comprise an HGF gene as an active ingredient, and methods that comprise the step of administering such an agent to a target site.

### Background Art

Hepatocyte growth factor (HGF) is a cytokine discovered as a factor that causes proliferation of hepatic parenchymal cells *in vitro* (Biochem. Biophys. Res. Commun. 122: 1450 (1984); Proc. Natl. Acad. Sci. USA 83: 6489 (1986); FEBS Letters 22: 231 (1987); Nature 342: 440-443 (1989); Proc. Natl. Acad. Sci. USA 87: 3200 (1991)). HGF is a plasminogen-related and mesenchyme-derived pleiotropic growth factor, and is known to regulate cell growth and cell motility in various types of cells (Nature 342: 440-443 (1989); Biochem. Biophys. Res. Commun. 239: 639-644 (1997); J. Biochem. Tokyo 119: 591-600 (1996)). HGF is also known to be an important factor regulating embryogenesis and morphogenic processes in the regeneration of a number of organs (Exp. Cell Res. 196: 114-120 (1991); Proc. Natl. Acad. Sci. USA 90: 1937-1941 (1993) ; Gene Therapy 7: 417-427 (2000)). HGF not only contributes *in vivo* as a liver regeneration factor in the repair and regeneration of damaged liver, it has also been shown to possess an angiogenic effect, and can play an important role in the treatment or prevention of ischemic or arterial diseases (Symp. Soc. Exp. Biol. 47 cell behavior 227-234 (1993); Proc. Natl. Acad. Sci. USA 90: 1937-1941 (1993); Circulation 97: 381-390 (1998)).

Since HGF displays a variety of functions, including angiogenic functions, as described above, various studies for its use in pharmaceutical agents have been carried out (Jikken Igaku (Experimental Medicine) (supplementary volume) 10(3): 330-339 (1992)). For example, there are reports of using HGF as an:
anticancer agent (Unexamined Published Japanese Patent Application No. (JP-A) Hei 6-25010);
therapeutic agent for lung injuries (JP-A Hei 6-172207);
agent for relieving the side effects of cancer therapy (JP-A Hei 6-340546);
agent for enhancing the growth of epithelial cells (JP-A Hei 7-179356);
agent for relieving the side effects of immunosuppressants (JP-A Hei 7-258111);
therapeutic agent for fulminant hepatitis (JP-A Hei 10-167982) ;
therapeutic agent for myocardial infarctions (JP-A Hei 11-246433);
therapeutic agent for arterial diseases (JP-A Hei 8-295634);
therapeutic agent for obesity (JP-A Hei 10-279500);
therapeutic agent for dilated cardiomyopathy (JP-A Hei 11-1439);
therapeutic agent for amyotrophic lateral sclerosis (JP-A 2002-87983);
preventive agent for pulmonary fibrosis (JP-A Hei 8-268906);
therapeutic agent for cartilage disorders (JP-A Hei 8-59502);
collagen degradation promoting agent (JP-A Hei 7-300426);
therapeutic agent for gastroduodenal diseases (JP-A Hei 7-138183);
therapeutic agent for cranial nerve disorders (JP-A Hei 7-89869);
therapeutic agent for acute renal failure (Published Japanese Translation of International Publication No. 2001-516358);
therapeutic agent for ischemic diseases/arterial diseases (WO 00/07615);
therapeutic agent for diabetes (WO 98/32458);
external agent for hair (JP-A Hei 5-213721);
dermal cosmetics (JP-A Hei 5-213733);
agent for promoting hair growth (JP-A Hei 5-279230);
agent for increasing megakaryocytes (JP-A Hei 7-101876);
differentiation-inducing agent (JP-A 2002-78482);
therapeutic agent for renal glomerular diseases (JP-A Hei 9-87199);
therapeutic agent for cachexia (JP-A Hei 10-316584);
therapeutic agent for multiple organ failure (JP-A Hei 10-310535);
therapeutic agent for ischemic diseases (WO 96/32960);
agent for cell proliferation and differentiation (Published Japanese Translation of International Publication No. Hei 10-503923) ;
agent for growth and differentiation of hematopoietic cells (Published Japanese Translation of International Publication No. Hei 10-509951);
agent for improving neuropathy (JP-A Hei 7-41429); and
therapeutic agent for hypoglycemia and glycogen diseases (JP-A Hei 10-7586).

Typically, proteinaceous formulations are administered intravenously. In ischemic disease models, HGF administration is exemplified by intravenous and intra-arterial administration (Circulation 97: 381-390 (1998)). Although intravenous or intra-arterial HGF administration has been shown to be effective in this type of animal model, no conclusions have been reached regarding effective methods of administration, dosages, and such of HGF for specific diseases. In particular, HGF' s short half-life in the blood has emerged as a problem when applying HGF proteins as pharmaceutical agents. Since the half-life of HGF in the blood is short as ten minutes, it has been difficult to maintain HGF concentration in the blood at a level where HGF functions sufficiently. Furthermore, another challenge has been raised regarding the delivery of an effective amount of HGF to affected sites.

Thanks to remarkable technological progress in the field of molecular biology, gene therapy involving the introduction of genes into cells is now possible. Gene therapy can generally be used in various medical treatments (Science 256: 808-813 (1992); Anal. Biochem. 162: 156-159 (1987)). Selecting an appropriate vector for gene transfer is particularly important for successful gene therapy. So far, vectors derived from viruses such as adenoviruses have been suggested for use in gene transfer.

However, viral vectors have also been suggested to potentially have the following dangers:
viral infection-associated toxicity;
development of viral pathogenicity associated with the depressed immunological function of the host;
mutagenic or carcinogenic nature of the viruses; and such.

As an alternative to methods using viral vectors, *in vivo* gene transfer methods using liposomes together with viral outer membranes, or HVJ-liposome-mediated gene transfer methods, have been developed (Science 243: 375-378 (1989) ; Anal. NY Acad. Sci. 772: 126-139 (1995)). *In vivo* gene transfers into various tissues, including the liver, kidney, vascular wall, heart, and brain, have been successfully accomplished using these methods (Gene Therapy 7: 417-427 (2000); Science 243: 375-378 (1989); Biochem. Biophys. Res. Commun. 186: 129-134 (1992); Proc. Natl. Acad. Sci. USA 90: 8474-8478 (1993); Am. J. Physiol. 271 (Regulatory Integrative Comp. Physiol. 40):R1212-R1220 (1996)).

However, since methods using HVJ-liposomes require different vehicles, such as viruses and liposomes, they are complicated to prepare. Furthermore, fusing HVJ virions with liposomes makes the average diameter of the resultant particles 1.3 times that of a viral particle. This increased diameter is known to reduce cell fusion activity to 10% or less of that of a wildtype virus, and there are tissues to which gene transfer is impossible or inefficient. Accordingly, a gene transfer method that uses viral envelope vectors was developed as a method that allows safer and more efficient gene therapy (Japanese Patent Application No. 2001-026185 / JP-A 2001-286282). In this method, an inactivated virus, which cannot replicate viral proteins, is used as a viral envelope, and genes are enclosed within this viral envelope. The vector can be used for gene transfer into cultured cells, biological tissues, and such. The use of such viral envelope vectors is known to enable safe and highly efficient gene transfer into the liver, skeletal muscle, uterus, brain, eyes, carotid arteries, skin, blood vessels, lungs, heart, kidneys, spleen, cancer tissues, nerves, B lymphocytes, respiratory organ tissue, suspended cells, and such.

Cerebrovascular disorders, represented by cerebral infarctions and intracerebral hemorrhages, are important diseases that are also socially significant. Although mortality due to cerebrovascular disorders has declined in recent years, such diseases are still highly ranked causes of death. Patients affected with the aftereffects of an ischemic cerebrovascular disorder, and hospitalized or outpatients being treated by clinical institutions, still continue to increase.

Generally, a cerebral infarction is a condition whereby the brain tissue becomes irreversibly necrotic, due to ischemic lesions caused by occlusion, or by a decrease of perfusion pressure in the cerebral artery or carotid artery. Cerebral infarctions can be categorized into following three major groups (Manabe, H., and Omae, T. Ed. "Nou-Kekkan Shogai (Cerebrovascular disorder)" Life Science Publishing, p54-55 (1992); Imura, H. *et al*. Ed., "Saishin Naikagaku Taikei Dai 66 Kan Nou-Kekkan Shogai (Integrated handbook of internal medicine, Vol. 66, Cerebrovascular disorder)" Nakayama Shoten, p28 (1996)):
(1) cerebral thrombosis, in which ischemic necrosis occurs in the cerebral tissue as a result of arterial occlusion, where the arterial occlusion is caused by increased blood viscosity, decreased perfusion pressure, or such, arising from sclerotic lesions in the cerebral artery;
(2) cerebral embolism, in which an embolus forms in the cerebral artery due to an intracardiac thrombus or, although rare, due to a thrombus detached from the arterial wall; and
(3) hemodynamic infarction, caused by reduced blood flow to the peripheral brain tissue due to the constriction or occlusion of the cephalic artery or intracranial cerebral artery.

In cerebral infarction, a cerebral edema occurs within a few hours of the onset of disease, and this condition continues for approximately one week after onset. Thereafter, the edema gradually decreases, but becomes fixed as an infarcted area within one to three months after onset. The cerebral edema causes the volume of the brain to increase. Since the brain is covered with a hard cranium, when the volume of the brain exceeds a certain limit due to the cerebral edema, rapid increase in tissue pressure and intra-cranial pressure results. Thus, brain damage worsens, and thereafter, the extent of the infarcted area is fixed (Inamura, K., Terashi, A. "Nippon Rinsho, Dai 51 Kan, CT, MRI Jidai no No-Sotchu Gaku, Jo-Kan (Nippon Rinsho, Vol. 51, Cerebral Apoplexy in the Age of CT and MRI, No. 1)" Nippon Rinsho, p231-239 (1993)). When an infarction occurs in a section of the brain, those functions carried by the affected part, such as cognition, perception, sense, and memory, are lost.

To date, nerve cells have been clinically recognized as being vulnerable to ischemia. Certain types of nerve cells are damaged when placed under ischemic conditions for only a few minutes, and these cells subsequently die. In hippocampal formations and pyramidal cells under ischemic conditions, a conduction block occurs after significant nerve excitation associated with depolarization. Subsequently, cell functions are lost due to the cytotoxicity caused by increased levels of extracellular glutamic acid, intracellular calcium ion, free radicals, and such, and the cells eventually die. If the irreversible changes caused by ischemia can be appropriately treated in an acute stage, it is thought that mortality rates can be improved, and aftereffects alleviated. Current treatment for cerebral infarction involves administering antiplatelet agents, agents for improving cerebral circulation and metabolism, and such. Among these antiplatelet agents, pharmaceutical agents effective for treating acute stage cerebral thrombosis exist. However, since these agents promote hemorrhagic cerebral infarction in patients suffering from cerebral hemorrhages or cerebral infarctions that develop symptoms similar to cerebral thrombosis, their use in these patients is contraindicated, and the disease type must be carefully diagnosed when using such agents.

During the acute stage, the use of agents for improving cerebral circulation used for administration during the chronic stage, which is approximately one month after the cerebral infarction attack, is considered unfavorable (Kameyama, M. Ed. "Nou-sotchu Chiryou Manyuaru (Treatment Manual of Cerebral Apoplexy)" Igaku-Shoin, p172-173 (1991)). Reperfusion therapy such as thrombolytic therapy, bypass surgery, thrombendarterectomy, and embolectomy are used as alternative methods of treatment during the hyperacute stage, in order to re-establish blood flow to regions where cells have not yet died. However, when the brain tissue is already irreversibly damaged by the cerebral infarction, the subsequent re-establishment of blood flow is problematic due to the danger of aggravating tissue injury, such as increasing the hemorrhagic infarction and cerebral edema (Okada, Y., "Shinkei Kenkyu no Shinpo (Progress in Neurologic Research)" Vol. 40, No. 4, p. 655-665, Igaku-Shoin, (1996); Takahasi, A. "medicina" Vol. 32, No. 11, p. 2261-2263, Igaku-Shoin, (1991)).

At present, the pharmaceutical agents used in the acute stages of cerebral infarctions are risky in that they may cause hemorrhagic infarctions and ischemic/reperfusion injuries. Furthermore, these agents are not completely satisfactory due to other problems such as the limited pathogenic conditions targeted, and the limited period over which administration is expected to be therapeutically effective.

### Disclosure of the Invention

An objective of the present invention is to provide pharmaceutical agents that can reduce the degree of brain injury caused by cerebral ischemic/reperfusion injuries associated with reperfusion after blood flow to the brain has been cut off. The HGF gene differs from other angiogenesis factors, such as VEGF, in that it does not increase the permeability of newly produced blood vessels. In cerebrovascular disorders in particular, increased vascular permeability increases the danger of injury to cerebral tissues by cerebral edema and increased intracerebral pressure. Thus, therapeutic and preventive methods using HGF that do not increase vascular permeability, are advantageous compared to methods using other angiogenesis factors.

An objective of the present invention is to provide therapeutic or preventive agents that use an HGF gene, against cerebrovascular disorders, and to provide methods of treating and preventing cerebrovascular disorders using these pharmaceutical agents. More specifically, the present invention is summarized as follows.
(1) An agent for treating or preventing a cerebrovascular disorder, wherein the agent comprises an HGF gene.
(2) The agent of (1), wherein the cerebrovascular disorder is a cerebral infarction.
(3) The agent of (1) or (2), wherein the agent is in the form of a tablet, pill, sugar-coated tablet, capsule, liquid, gel, ointment, syrup, slurry, or suspension.
(4) The agent of any one of (1) to (3), wherein the agent is used to transfer a gene into a cell by a method employing a viral envelope vector, internal type liposome, electrostatic type liposome, HVJ-liposome, or improved HVJ-liposome, a receptor-mediated gene transfer method, a method for transferring a nucleic acid molecule along with a carrier into a cell by using a particle gun, a direct introduction method using a naked-DNA, or an introduction method using a cationic polymer.
(5) The agent of any one of (1) to (3), wherein the agent is used to transfer a gene into a cell by employing an HVJ-envelope.
(6) A method for treating or preventing a cerebrovascular disorder, wherein the method comprises the step of introducing an HGF gene into a mammal.
(7) The method of (6), wherein the cerebrovascular disorder is a cerebral infarction.
(8) The method of (6) or (7), wherein the method comprises the step of introducing an HGF gene into a mammal two to three times by employing an HVJ-envelope.
(9) Use of an HGF gene to produce an agent for treating or preventing a cerebrovascular disorder.
(10) The use of the HGF gene of (9), wherein the cerebrovascular disorder is a cerebral infarction.

The term "HGF gene" as used in this invention refers to a nucleic acid molecule that can express an HGF (an HGF protein). Herein, the term "nucleic acid molecule" refers to molecules such as DNAs, RNAs, cDNAs, and mRNAs. Specifically, cDNAs that encode HGF are described in, for example, Nature 342: 440 (1989), Patent No. 2777678, and Biochem. Biophys. Res. Commun. 163: 967 (1989). The nucleotide sequences of cDNAs encoding HGF are described in the aforementioned literature, and are also registered in databases such as GenBank. Based on this sequence information, cDNAs of HGF can be cloned by using appropriate sequence segments as PCR primers, and by performing RT-PCR using, for example, mRNAs derived from the liver or leukocytes. One skilled in the art can readily perform such cloning by following fundamental texts, such as Molecular Cloning 2nd edition (Cold Spring Harbor Laboratory Press) (1989). Furthermore, by screening genomic DNA libraries, genomic DNAs can be isolated.

The HGF genes of this invention are not limited to these cDNAs and genomic DNAs. As long as a gene encodes a protein that, when expressed, has practically the same function as HGF, the gene can be used as an HGF gene of this invention. More specifically, the HGF genes that can be used in this invention encompass 1) nucleic acids that hybridize under stringent conditions with an aforementioned cDNA, or 2) nucleic acids encoding proteins that comprise an amino acid sequence of a protein encoded by an aforementioned cDNA, in which one or more (preferably several) amino acids are deleted, substituted, added, and/or inserted, so long as the proteins encoded by such nucleic acids have a function of HGF. The nucleic acids of the aforementioned 1) and 2) can be readily obtained by methods such as site-directed mutagenesis, PCR methods (see, Current Protocols in Molecular Biology edit. Ausubel *et al*. (1987) John Wiley & Sons, Sections 6.1-6.4), or conventional hybridization methods (see, Current Protocols in Molecular Biology edit. Ausubel *et al*. (1987) John Wiley & Sons, Sections 6.3-6.4).

More specifically, by using a known sequence of an HGF gene, or a portion thereof as a probe; or by using an oligonucleotide that specifically hybridizes with that DNA sequence as a primer, those skilled in the art can isolate nucleic acids that hybridize with that DNA sequence. Stringent hybridization conditions for obtaining nucleic acids that encode proteins functionally equivalent to known HGF are normally "1x SSC at 37°C" and such, more stringently "0.5x SSC and 0.1% SDS at 42°C" and such, and even more stringently "0.1x SSC and 0.1% SDS at 65°C" and such. When more stringent hybridization conditions are used, nucleic acids comprising sequences more homologous to the probe sequence can be isolated. However, the SSC, SDS, and temperature conditions recited herein are only examples. One skilled in the art can readily set conditions that constitute the same degree of stringency as the above-described, by considering the above-mentioned conditions and other conditions that determine the stringency of hybridization, such as probe concentration, probe length, and reaction time.

The amino acid sequences of the proteins encoded by the nucleic acids isolated by the above-mentioned hybridization methods or PCR methods usually show high homology to conventionally known HGF proteins. The term "high homology" refers to a sequence homology of at least 50% or more, more preferably 70% or more, even more preferably 90% or more (for example, 95% or more). The sequence identity of amino acid sequences and nucleotide sequences can be determined using Karlin and Altschul's BLAST algorithm (Proc. Natl. Acad. Sci. USA 90: 5873-5877 (1993)). Programs such as BLASTN and BLASTX have been developed based on this algorithm (Altschul *et al*. J. Mol. Biol. 215: 403-410 (1990)). When nucleotide sequences are analyzed using BLASTN, based on BLAST, parameters are set, for example, at score = 100 and wordlength = 12. When amino acid sequences are analyzed by using BLASTX, based on BLAST, parameters are set, for example, at score = 50 and wordlength = 3. When using the BLAST and Gapped BLAST programs, the default parameters for the respective programs are used. Specific techniques for these analytical methods are well known (http://www.ncbi.nlm.nih.gov).

As described above, the HGF genes used in this invention can be naturally occurring or artificial nucleic acids, as long as the proteins encoded by the genes comprise HGF activity. HGF has the activity of promoting the *in vitro* proliferation of hepatic parenchymal cells. Therefore, it is possible to determine whether the proteins encoded by the nucleic acids obtained by the above-described hybridization methods or such, or the proteins encoded by the nucleic acids of a modified naturally occurring HGF gene, have HGF activity, by, for example, investigating the effect of these proteins on the *in vitro* proliferation of hepatic parenchymal cells.

The gene transfer methods, transfer forms, transfer amounts, and such used in the gene therapies of this invention are described below.

Methods for administering gene therapy agents comprising an HGF gene as an active ingredient can be classified into two groups: methods using non-viral vectors; and methods using viral vectors. The preparation methods, administration methods, and such for these vectors are described in detail in experiment manuals (Jikken Igaku (Experimental Medicine) Supplementary Volume, "Idenshichiryo no Kisogijyutsu (Fundamental Techniques for Gene Therapy)", Yodosha, 1996; Jikken Igaku (Experimental Medicine) Supplementary Volume, "Idenshidonyu & Hatsugenkaiseki Jikkenho (Experimental Methods for Gene Transfer & Expression Analysis)", Yodosha, 1997; "Idenshi-chiryo Kaihatsu Kenkyu Handbook (Handbook of Gene Therapy Research and Development)", Nihon Idenshichiryo Gakkai (The Japan Society of Gene Therapy) Edition, NTS, 1999). Such vectors and methods are specifically described below.

A recombinant vector, where a target gene has been inserted into a conventional non-viral gene expression vector, can be used to introduce a target gene into cells and tissues by the methods shown below.

Examples of methods for transferring genes into cells are: lipofection methods, calcium-phosphate co-precipitation methods, DEAE-dextran methods, methods of direct infusion of DNA using a glass capillary tube, etc. Methods for transferring genes into tissues include methods using virus envelope vectors, internal type liposomes, electrostatic type liposomes, HVJ-liposomes, improved type HVJ-liposomes (HVJ-AVE liposomes), receptor-mediated gene transfer methods, methods for transferring carriers (such as metal particles) along with DNAs using particle guns, methods for directly introducing naked-DNAs, introduction methods using positively charged polymers, etc.

The aforementioned HVJ-liposomes are constructed by incorporating a DNA into a liposome formed by a lipid bilayer, then fusing this liposome with an inactivated Sendai virus (hemagglutinating virus of Japan; HVJ). The use of HVJ-liposomes is characterized by extremely high cell membrane fusion compared to conventional liposome methods, and is one of the especially preferred forms of introduction. Methods for preparing HVJ-liposomes are described in detail in, for example, Experimental Medicine Supplementary Volume, "Idenshichiryo no Kisogijyutsu (Fundamental Techniques of Gene Therapy)", Yodosha, 1996; Experimental Medicine Supplementary Volume, "Idenshidonyu & Hatsugenkaiseki Jikkenho (Experimental Methods for Gene Transfer & Expression Analysis)", Yodosha (1997); J. Clin. Invest. 93: 1458-1464 (1994); Am. J. Physiol. 271: R1212-1220(1996). Methods for using the HVJ-liposome are described in, for example, Molecular Medicine 30: 1440-1448 (1993); Jikken Igaku (Experimental Medicine), 12: 1822-1826 (1994); and Tanpakushitsu Kakusan Kouso (Protein, Nucleic Acid, and Enzyme), 42, 1806-1813 (1997) ; and preferably described in Circulation 92 (Suppl. II): 479-482 (1995).

Furthermore, methods using viral envelopes are particularly preferred methods for administering an HGF gene of this invention. Viral envelopes can be prepared by mixing a purified virus with an expression vector of interest in the presence of a surfactant, or by freezing and thawing a mixture of a virus and an expression vector (Japanese Patent Application No. 2001-026185 / JP-A 2001-286282).

The viruses that can be used in the methods using viral envelopes are viruses belonging to families such as the retrovirus, togavirus, coronavirus, flavivirus, paramyxovirus, orthomyxovirus, bunyavirus, rhabdovirus, poxvirus, herpesvirus, baculovirus, and hepadnavirus families, and are especially preferably HVJs. Herein, these viruses can be either wild-type or recombinant viruses. In particular, a recombinant HVJ reported by Hasan, M. K. *et al*. (J. General Virol. 78: 2813-2820 (1997)), Yonemitsu, Y. *et al*. (Nature Biotech. 18: 970-973 (2000)), or such may be used as an HVJ.

While the Z strain (available from ATCC) of HVJ is generally preferable for use in the methods using HVJ-liposomes or HVJ-envelopes, fundamentally, other HVJ strains (for example, ATCC VR-907 and ATCC VR-105) can also be used. In the methods for preparing a viral envelope, purified viruses can be inactivated by UV irradiation and such, and then mixed with a desired expression vector. Surfactants that may be used for mixing the virus and expression vector are, for example, octylglucoside, Triton X-100, CHAPS, and NP-40. Viral envelope vectors prepared in this manner can be introduced by injection or such into tissues to be targeted for therapy or disease prevention. Furthermore, by freezing at -20°C, the viral envelope vectors can be stored for at least two to three months.

The expression vectors that may be used herein can be any expression vectors, so long as they can express a desired gene *in vivo.* Examples of the expression vectors are pCAGGS (Gene 108: 193-200 (1991)), pBK-CMV, pcDNA3.1, and pZeoSV (Invitrogen, Stratagene)

Representative methods for gene transfer with viral vectors are those methods using viral vectors such as recombinant adenoviruses and retroviruses. More specifically, a subject gene can be transferred into cells by the steps of: introducing the gene into DNA or RNA viruses such as detoxicated retroviruses, adenoviruses, adeno-associated viruses, herpesviruses, lentiviruses, vaccinia viruses, poxviruses, polioviruses, sindbis viruses, Sendai viruses, SV40, or human immunodeficiency viruses (HIV) (see Pharmacol. Ther. 80: 35-47 (1998); Front. Biosci. 4: E26-33 (1999); J. Recep. Signal. Transduct. Res. 19: 673-686); and then infecting cells with the resultant recombinant virus.

The infection efficiency of adenovirus vectors is much greater than the other aforementioned viral vectors. Thus, from this viewpoint, the use of an adenovirus vector system is preferred.

Methods for introducing an agent of the present invention during gene therapy include: the *in vivo* introduction of a gene therapy agent directly into the body; and the *ex vivo* introduction of a gene therapy agent into a cell harvested from the body, followed by reintroduction of the modified cell into the body (Nikkei Science, April 1994, 20-45; Gekkann Yakuji 36 (1), 23-48, 1994; Jikken Igaku (Experimental Medicine) Supplementary Volume, 12 (15), 1994; "Idenshi-chiryo Kaihatsu Kenkyu Handbook (Handbook of Gene Therapy Research and Development)", Nihon Idenshichiryo Gakkai eds. (The Japan Society of Gene Therapy) Edition, NTS, 1999). *In vivo* methods are particularly preferred in the present invention.

Various formulations, (for example, liquid preparations), suited to each of the above-mentioned administration methods may be adopted as the form of the preparations. For example, an injection comprising a gene as an active ingredient can be prepared by conventional methods, which might include dissolving a gene in an appropriate solvent (e.g. a buffer solution, such as PBS, physiological saline, and sterilized water), sterilizing by filtration as necessary, and then loading into a sterile container. Conventional carriers or such may be added to injection agents as required. Alternatively, liposome preparations, such as preparations comprising HVJ-liposome, can be prepared as suspensions, frozen agents, or centrifugally concentrated frozen agents.

For the therapeutic or preventive agents of this invention, an HGF gene can be used as the single active ingredient, and can also be used together with other known factors having angiogenic functions. For example, factors such as VEGF and EGF have been reported to possess angiogenic functions, thus genes encoding these factors may be concurrently applied. Furthermore, since growth factors such as EGF have been reported to repair cell lesions in various tissues, genes encoding a variety of growth factors may be concurrently used as necessary. From the description of the present invention, it is clear to those skilled in the art that the following agents can be used, as required, in combination with the HGF gene in the therapeutic or preventive agents of the present invention: other pharmaceutical agents having therapeutic or preventive effects against a cerebrovascular disorder to be treated or prevented; and substances that stabilize or enhance HGF (for example, heparin-like substances (JP-A Hei 10-158190) and oligosaccharides (JP-A Hei 5-301824)). If these additional pharmaceutical agents and substances are encoded by genes, the genes encoding them may be administered together with the HGF gene in the therapeutic or preventive agents of this invention.

Appropriate administration methods and sites to be administered can be selected for the therapeutic and preventive agents of the present invention depending on the disorders, symptoms, or such to be treated. Parenteral administration is particularly preferred. The cisterna magna and lumbar spine are especially preferable administration sites. The cisterna magna or lumbar spine is punctured into the meningeal, and then an appropriate amount of spinal fluid is collected to confirm the puncture site, and to prevent an increase in intracranial pressure. The therapeutic or preventive agent is then administered. For example, a method for administering HVJ-liposome complex using a cannula, reported by Hayashi, K. *et al*. (Gene Therapy 8: 1167-73 (2001)), could be applied to administer the therapeutic or preventive agents of this invention into the cisterna magna.

The effect of the therapeutic or preventive agents of this invention is considered to be caused by using HGF gene administration to sustain neurons by suppressing apoptosis in the so-called "penumbra" region around the focus of the cerebral infarction. Therefore, "therapy" in the present invention means treatment to reduce the effect of a blood flow disorder after it occurs in the brain.

More specifically, a therapeutic effect of the pharmaceutical agents or methods of this invention refers to an effect whereby the administration of a pharmaceutical agent or the application of a method of this invention, after the onset of cerebrovascular disorder, reduces brain tissue damage caused by the cerebrovascular disorder, compared to when nothing is administered. Therefore, "therapy" in the present invention encompasses not only complete recovery from the damage, but also the effect of reducing the degree of damage.

On the other hand, "prevention" in the present invention refers to a reduction in the effect of a blood flow disorder, by the preventative administration of an HGF gene prior to the blood flow disorder occurring in the brain. More specifically, HGF gene administration is said to have a preventive effect when the administration of an HGF gene prior to the onset of a cerebrovascular disorder, such as a cerebral infarction, reduces the brain tissue damage caused by the cerebrovascular disorder that occurs after administration, compared to when nothing is administered. Therefore, "prevention" in the present invention encompasses not only complete recovery from the damage, but also the effect of reducing the degree of damage.

The terms "therapeutic agent" and "preventive agent" of this invention are used as terms that mean pharmaceutical formulations comprising the above-mentioned functions. The therapeutic methods and preventive methods of this invention are methods comprising the step of administering a pharmaceutical formulation comprising an above-mentioned effect.

On the other hand, cerebrovascular disorders in this invention refer to conditions in which blood flow to the brain is inhibited. Disorders that cause inhibition of blood flow to the brain are, for example, cerebral infarctions and intracerebral hemorrhages. Inhibited blood flow is not limited to that caused by disease. For example, conditions of reduced blood flow resulting from a blood vessel artificially sealed off in surgical treatment, and from an injured blood vessel due to a wound, are included in the cerebrovascular disorders of this invention. The cerebrovascular disorders of this invention include, for example, cerebrovascular disorders that cause ischemic or infarcted lesions in the cerebral parenchyma, such as a cerebral infarction (cerebral thrombosis, cerebral embolism, and the like), intracerebral hemorrhage, subarachnoid hemorrhage, hypertensive encephalopathy, cerebrovascular dementia, and Alzheimer-type dementia.

The therapeutic or preventive agents of the present invention comprise a sufficient amount of HGF gene to accomplish the objective intended by the pharmaceutical agent. In other words, the agents of the present invention comprise an HGF gene in a "therapeutically effective amount" or a "pharmacologically effective amount". The terms "therapeutically effective amount" and "pharmacologically effective amount" are effective amounts of pharmaceutical agent to produce the intended pharmacological results, and sufficient amounts to relieve the symptoms of the patient to be treated. Assays useful in confirming an effective dose for a particular application are, for example, methods for measuring the degree of recovery from a target disease. The amount that should actually be administered varies depending on the age, weight, and symptom of the patient being treated, as well as on the administration method and such, and is preferably an amount optimized to achieve a desired effect without marked side effects.

Therapeutically effective amounts, pharmacologically effective amounts, and toxicity can be determined by cell culture assays or optionally, by using appropriate animal models. Such animal models can be used to determine the desired concentration range and administration route for a pharmaceutical agent. Based on these animal models, one skilled in the art can determine the effective dose for a human. The dose ratio of therapeutic effect to toxic effect is called the "therapeutic index", and this can be expressed as the ratio: ED50/LD50. Pharmaceutical compositions with a large therapeutic index are preferred. An appropriate dose is selected according to the dosage form, the patient's sensitivity, age, and other conditions, and the type and severity of the disease. Although the dose of a therapeutic agent of the present invention differs depending on the condition of the patient, the adult dose of an HGF gene is in the range of approximately 1 µg to approximately 50 mg, preferably in the range of approximately 10 µg to approximately 5 mg, and more preferably in the range of approximately 50 µg to approximately 5 mg. In particular, since an HGF gene can be repeatedly administered by an HVJ envelope method, administration of the HGF gene can be performed not only once, but many times, such as two or three times, to obtain better therapeutic or preventive effects. Such multiple administrations using an HVJ envelope are also included in the therapeutic or preventive methods of this invention.

### Brief Description of the Drawings

Fig. 1 is a set of photographs of the TTC-stained coronal slices of rats (three out of six animals) belonging to the physiological saline group.
Fig. 2 is a set of photographs of the TTC-stained coronal slices of rats (three out of six animals) belonging to the pVAXI group.
Fig. 3 is a set of photographs of the TTC-stained coronal sections of rats (three out of six animals) belonging to the HGF group.
Fig. 4 is a graph comparing the areas of the infarcted areas in the physiological saline group, the pVAXI group, and the HGF group. A schematic diagram of the rat brain is shown above right. The coronal sections were prepared by slicing the brains along the lines indicated as 1 to 5. The numbers 1 to 5 in the schematic diagram correspond to those on the horizontal axis. The vertical axis indicates the percentage (%) of the total coronal slice area that is occupied by the infarcted area.

### Best Mode for Carrying out the Invention

Hereinafter, the present invention will be specifically illustrated with reference to Examples, but is not to be construed as being limited thereto.

### (1) Production of HGF gene expression vector

Human HGF cDNA (2.2 kb) was inserted between the BamHI and NotI sites of the pVAX1 vector (Invitrogen).

### (2) Preparation of the HVJ-envelope

Immediately prior to its use, the purified Sendai virus (HVJ) (Z strain) was inactivated by UV irradiation (99 mJ/cm²). Next, the inactivated HVJ (15, 000 hemagglutination units (HAU)) and the vector produced in (1), or an expression vector not comprising HGF cDNA, were mixed with 0.3% Triton X-100 solution at 4°C. This mixture was centrifuged for 15 minutes at 4°C. After removing the supernatant, buffering salt solution was added to the residue and then centrifuged for another 15 minutes at 4°C. After removing the supernatant, the pellet was suspended in 100 µL of phosphate-buffered saline, and used to investigate the effect on cerebral infarction.

### (3) Administration of HVJ envelope-DNA complex to a rat right middle cerebral artery occlusion model

Wistar rats weighing 250 to 270 g were anesthetized with halothane (4% at the time of introduction; maintained at 1%). Animals were then cisternally administered, using a 26 G needle, with: 1) physiological saline (100 µL) for the physiological saline group; 2) pVAXI (400 µg)/HVJ-E (15,000 HAU) (100 µL) for the pVAXI group, comprising vectors only; and 3) pVAXI-HGF (400 µg) /HVJ-E (15, 000 HAU) (100 µL) for the HGF-administered group. Six animals were used in each group. Three days after administration, 21 mm of 4-0 nylon suture coated with poly-L-lysine was inserted from the right external carotid artery into the right internal carotid artery under halothane anesthesia, to produce a right middle cerebral artery occlusion model. During this treatment, the body temperatures of the animals were maintained at around 37°C using a heating pad, and their blood pressures were measured at the caudal artery. Neurological evaluation was carried out one hour and 24 hours after this treatment. Neurological evaluation was performed according to the standard shown below, and the total scores of each group were compared (denoted in Table 1 as the Neurological Severity Score (NSS)). The results were statistically analyzed using a Mann-Whitney U test with StadtView 5.0J.
1) Presence of flexion of the left foreleg
- 0: Not flexed
- 0.5: Slightly flexed
- 1: Completely flexed

2) Resistance against lateral compression
- 0: Same degree of resistance to the right and left
- 0.5: Slightly weakened resistance
- 1: No resistance

3) Body Posture
- 0: Normal
- 0.5: Slightly bent to the left
- 1: Completely bent to the left

4) Position of the left foreleg
- 0: Quickly returns to original position
- 0.5: Returns to original position
- 1: Does not return to original position

5) Position of the right foreleg
- 0: Quickly returns to original position
- 0.5: Returns to original position
- 1: Does not return to original position

**Table 1**

| | HGF/HVJ | pVAXI/HVJ | Physiological p-Value saline |
|---|---|---|---|
| Body weight | 250.4 ± 2.0 | 253 ± 2.3 | 251.8 ± 2.4 |
| Blood pressure (before treatment) | 88.6 ± 1.9 | 88.8 ± 3.7 | 88.5 ± 2.1 |
| Blood pressure (after treatment) | 82.4 ± 1.7 | 81.2 ± 3.8 | 82.8 ± 1.4 |
| Body temperature (before treatment) | 37.4 ± 0.1 | 37.3 ± 0.1 | 37.2 ± 0.2 |
| Body temperature (after treatment) | 37.6 ± 0.1 | 37.1 ± 0.2 | 37.4 ± 0.2 |
| NSS (1 hr) | 1.0 ± 0.2 | 1.3 ± 0.4 | 1.4 ± 0.2 p<0.05* |
| NSS (24 hr) | 1.0 ± 0.1 | 1.4 ± 0.2 | 1.3 ± 0.3 p<0.05* |

| | | | |
|---|---|---|---|
| *Compared to the physiological saline group using a Mann-Whitney U test. | | | |

As shown in Table 1, differences in blood pressure and body temperature during the operation were not observed between the physiological saline group, pVAXI group, and HGF group. The score for neurological evaluation was significantly lower for the HGF group than the other two groups, suggesting that administration of the HGF gene maintains neurological function.

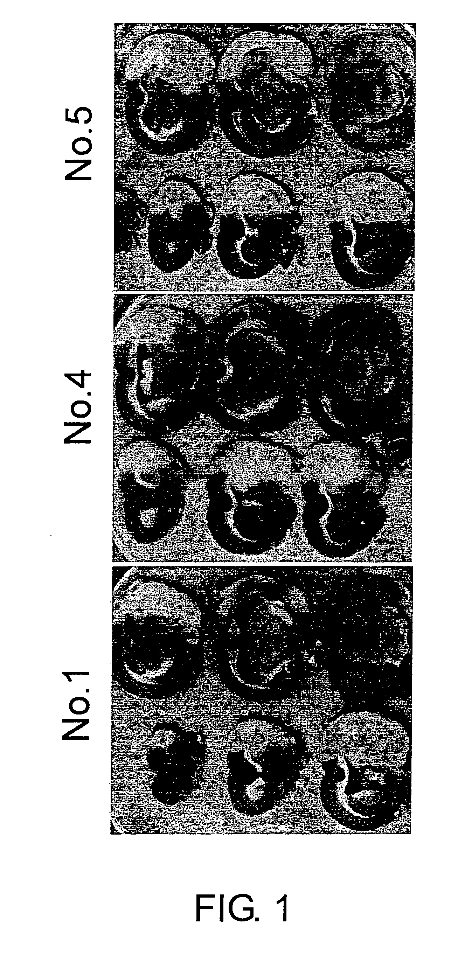

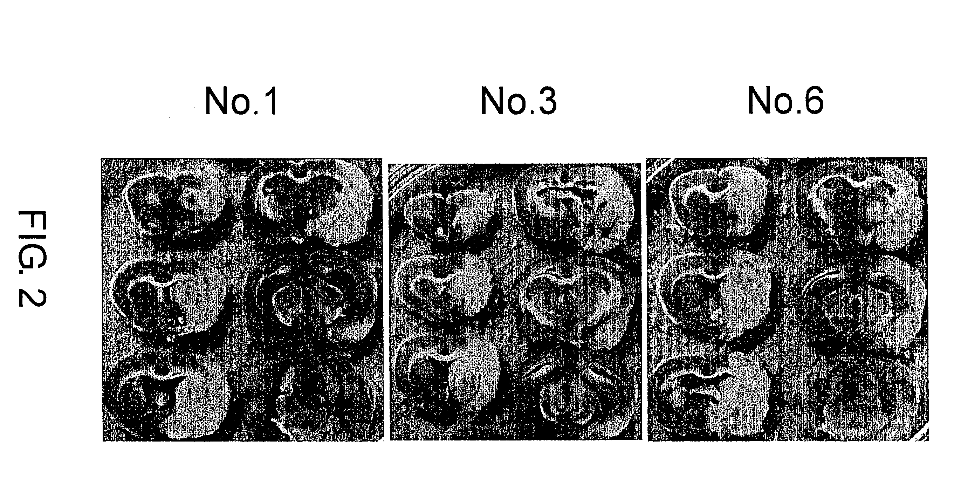

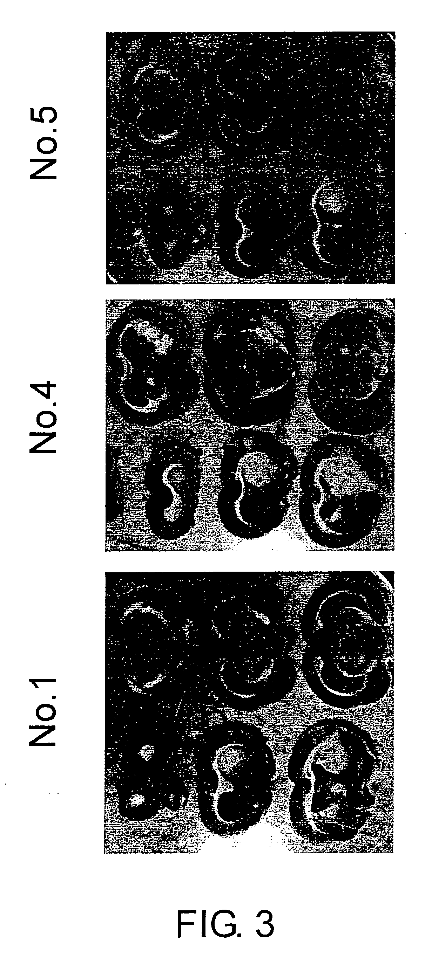

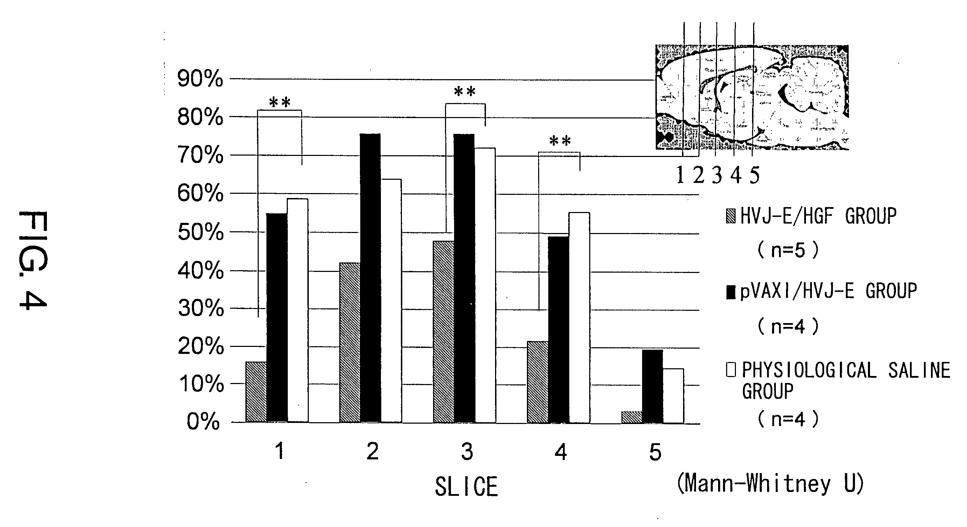

The animals were sacrificed 24 hours later, and coronal sections were prepared every 2 mm from the frontal pole (at the positions indicated by lines 1 to 5 in the schematic diagram to the top right of Fig. 4). TTC staining was performed, and the areas of the infarcted areas were compared (Figs. 1 to 3). To compare these areas, the numbers of pixels occupied by the infarcted areas were measured using Adobe Photoshop 5.0. The effect of the edema was taken into consideration, and evaluations were made according to the following formula: (the lateral area of a healthy brain - (the lateral area of an infarcted brain - area of infarcted area))/the lateral area of a healthy brain x 100 (%). Fig. 4 shows the results. The proportion of the infarcted area compared to the total area of the coronal section is indicated as a percentage. As is apparent from Figs. 1 to 4, the infarcted area in the HGF gene transfected group was confirmed to be reduced.

### Industrial Applicability

The above-mentioned results confirmed that the administration of an HGF gene shows advantageous effects, maintains neuronal function, and reduces the size of an infarcted area in the early stages of cerebrovascular disorders, such as cerebral infarctions. More specifically, HGF was shown to probably play a role in regulating cerebrovascular disorders. The present invention provides novel methods for treating cerebrovascular disorders, including cerebral infarctions, where the methods involve overexpressing HGF by introducing an HGF gene. The present methods utilizing HGF gene enable active treatment of cerebrovascular disorders, including cerebral infarction, by gene transfer. The present methods also enable maintenance of neuronal function and inhibition of the infarcted area in patients for whom appropriate treatment methods did not exist until now.

## Claims

1. An agent for treating or preventing a cerebrovascular disorder, wherein the agent comprises an HGF gene.

2. The agent of claim 1, wherein the cerebrovascular disorder is a cerebral infarction.

3. The agent of claim 1 or 2, wherein the agent is in the form of a tablet, pill, sugar-coated tablet, capsule, liquid, gel, ointment, syrup, slurry, or suspension.

4. The agent of any one of claims 1 to 3, wherein the agent is used to transfer a gene into a cell by a method employing a viral envelope vector, internal type liposome, electrostatic type liposome, HVJ-liposome, or improved HVJ-liposome, a receptor-mediated gene transfer method, a method for transferring a nucleic acid molecule along with a carrier into a cell by using a particle gun, a direct introduction method using a naked-DNA, or an introduction method using a cationic polymer.

5. The agent of any one of claims 1 to 3, wherein the agent is used to transfer a gene into a cell by employing an HVJ-envelope.

6. A method for treating or preventing a cerebrovascular disorder, wherein the method comprises the step of introducing an HGF gene into a mammal.

7. The method of claim 6, wherein the cerebrovascular disorder is a cerebral infarction.

8. The method of claim 6 or 7, wherein the method comprises the step of introducing an HGF gene into a mammal two to three times by employing an HVJ-envelope.

9. Use of an HGF gene to produce an agent for treating or preventing a cerebrovascular disorder.

10. The use of the HGF gene of claim 9, wherein the cerebrovascular disorder is a cerebral infarction.
